# EUROPEAN PATENT SPECIFICATION

(11) **EP 1 518 497 B1**
(45) Date of publication and mention of the grant of the patent: **08.08.2007**
(21) Application number: 04468017.1
(22) Date of filing: 21.09.2004
(51) Int. Cl.: A61B 5/103, A61H 39/02

(54) **Device for inspection of diabetic foot**
Vorichtung zur Inspektion des diabetischen Fusses
Dispositif d'inspection du pied diabétique

(30) Priority: 24.09.2003 SI 200300243 P
(43) Date of publication of application: 30.03.2005
(73) Proprietor: Dabic, Obrad, 1231 Ljubljana-Crnuce (SI)
(72) Inventor: Dabic, Obrad, 1231 Ljubljana-Crnuce (SI)
(74) Representative: Borstar, Dusan

(56) References cited:
- MASSACHUSETTS GUIDELINE FOR ADULT DIABETES CARE - DIABETES GUIDELINES WORK GROUP: "Foot Inspection and Monofilament Use Guide" May 2003 (2003-05), , XP002309858 Retrieved from the Internet: URL:http://www.maclearinghouse.com/PDFs/Di abetes/GuidelinesFY03/Foot_inspection.pdf> [retrieved on 2004-12-09] * the whole document *
- D. ARMSTRONG ET AL.: "Diabetic Foot Ulcers: Prevention, Diagnosis and Classification" 15 May 1998 (1998-05-15), AMERICAN ACADEMY OF FAMILY PHYSICIANS , XP002309859 Retrieved from the Internet: URL:http://www.aafp.org/afp/980315ap/armst ron.html> [retrieved on 2004-12-09] * abstract; figure 1 *

## Description

The subject matter of the invention is a device for diagnosing the impairments of the peripheral nervous system on the feet of diabetic patients.

The device enables precise, quick and reliable identification of parts of the foot where the nerves have been damaged or are completely dysfunctional.

The technical problem that the proposed invention resolves is the design and construction of a device which enables precise diagnosing of the aforesaid condition.

In the simplest method of foot examination the doctor, or nurse, touches the critical zones on the foot with cotton wool or specially prepared micro stick (monofilament), applying a force of 5 - 10 g. As he touches the patient's foot the doctor asks him if he feels the touch and where he feels it. The patient usually looks at what the doctor is doing and often gets confused, does not know what he is expected to say (because of pre-examination anxiety among other things). Formerly the examination was carried out with the patient answering the doctors questions seated behind a folding screen.

The fact that the patient is asked several times if and where he feels the touch makes it difficult for him to give a precise answer. As a result, the doctor himself is not absolutely sure and might make a wrong diagnosis which, in the long run, might lead to amputation of the affected foot.

Foot inspection by using a monofilament as such is known to those skilled in the art and is e.g. described in MASSACHUSETTS GUIDELINE FOR ADULT DIABETES CARE - DIABETES GUIDELINES WORK GROUP: »Foot inspection and Monofilament Use Guide«, may 2003 (2003-05), XP002309858, which is currently retrievable from the internet (URL:http://www.maclearinghouse.com/PDFs/Diabetes/GuidelinesFY03/Foot inspection.pdf).

This document discloses a method of inspecting a diabetic foot where a diagram of the foot is used which shows sites to be tested. In this procedure, the patient is required to look away or close his eyes. None of the prior art discloses a procedure where the answer of the patient resides in pointing to a specific foot aera on a foot diagram which corresponds to the aera that the diagnostician has touched. The proposed diabetic foot examination device is particularly adapted to facilitate this procedure.

The proposed diabetic foot examination Device consists of a semitransparent screen onto which is fastened the panel with pictures of the feet. The device also includes a lamp.

The invention will be explained in a greater detail on the basis of the enclosed drawings, where
Fig. 1 is a top view of a screen;
Fig. 2 is a cross-section of a screen along the plane A - A according to Fig. 1;
Fig. 3 is a longitudinal cross-section of the first embodiment of the lamp; and
Fig. 4 is a longitudinal cross-section of the second embodiment of the lamp.

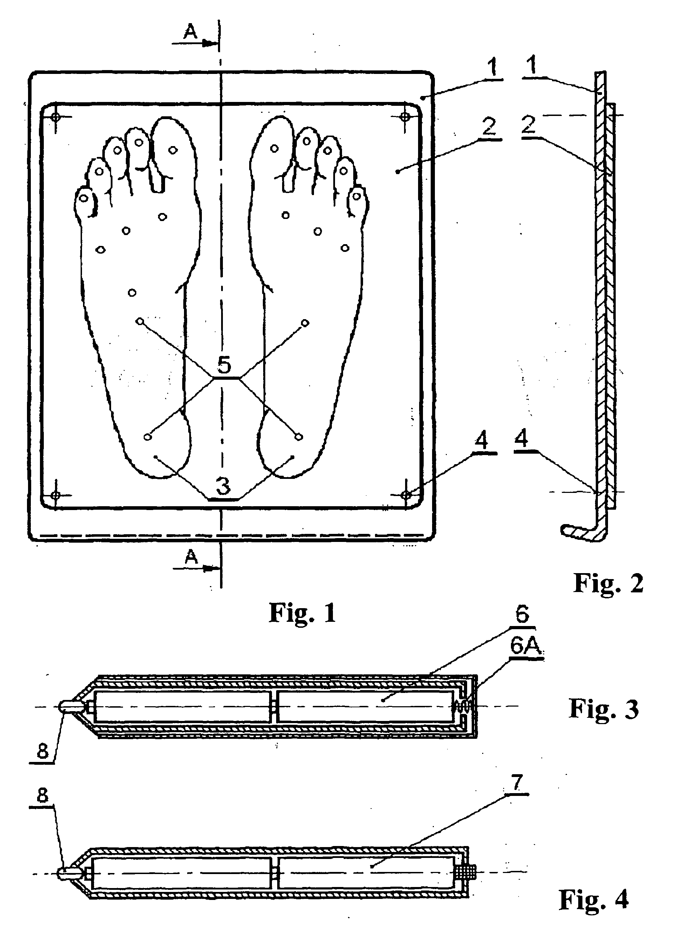

The diabetic foot examination device shown in Figs 1 to 4 is composed of a semitransparent screen 1, the panel 2 with pictures 3 of the feet, on which are marked the critical sites 5 to be tested.

The screen 1, and the panel 2 are joined with joining elements 4. The touch-screen lamp 6 has a flexible tube 6A, and a light emitting diode (LED) 8. The set may also include a classical lamp 7, fitted with a light emitting diode 8. The light emitting diode is used to prevent very bright light of the lamp 6, 7 from hurting the diagnostician's eyes.

The examination of the diabetic feet according to the device of the proposed invention is simple. The patient sits in a chair holding the screen 1 on his knees with one hand and the touch-screen lamp 6 in the other hand. The screen 1 stands between the patient and the diagnostician so that the patient cannot see what the diagnostician is doing. When the diagnostician touches a particular point on the patient's foot with the monofilament applying the force of 5 to 10 g, the patient whose peripheral nervous system is not impaired will fill the touch.

The patient responds to the touch by indicating with the lamp on the screen the point on the foot which the diagnostician has touched.

If the patient does not respond to the touch, the reason may be an impairment of the peripheral or the central nervous system. Such patient may have had many bouts of hypoglicaemia. Frequent bouts of deep hypoglicaemia are known to irretrievably damage grey cells, a result of which is that the patient cannot even perform the simplest operations, e.g. gently press the screen 1 with the lamp 6 to cause the light to appear.

When such a patient is being examined, he will be given an ordinary lamp 7 which will be switched on prior to the examination and will remain on till the end of the examination.

In such cases the diagnostician must pay even greater attention to the patient's movements.

The advantages offered by the Device include:
- The examination takes by far less time to previous methods of examination;
- the reliability of the method is great, for the diagnostician learns with certainty of the patient has or has not an impairment.
- the method works even if diagnostician and patient do not speak the same language.
- that device is indispensable in the examination of deaf mutes and persons who have suffered apoplexy.
- the patient concentrates on the expected excitation and is looking at the screen uninhibited by the presence the diagnostician.

The price of the Device is relatively cheap since most of it is made of cheap plastics and standard parts.

## Claims

1. Device for examination of a diabetic foot, comprising at least a monofilament, which is intended for successively pressing towards each desired zone on the foot of the patient in order to determine, whether the pressure and/or location thereof is then identified by the patient or not, **characterized in that** in addition to said monofilament said device also includes
- a semi-transparent screen (1), onto which is tastened a panel (2) with pictures (3) of the feet. and adapted to be held during examination either by the patient or his assistant in a desired position between the patient and the examiner, preferably on the knees of the patient;and
- a lamp (6, 7), preferably a diode (8), which is adapted to be held by the patient during the examination.

## Patentansprüche

1. Vorrichtung zur Untersuchung eines Fußes eines diabetischen Patienten, umfassend mindestens ein Monofilament, das dazu bestimmt ist, nacheinander auf jeden erwünschten Bereich auf dem Fuß des Patienten zu drücken, um zu bestimmen, ob der Druck und/oder der Ort davon vom Patienten identifiziert wird oder nicht, **dadurch gekennzeichnet, dass** die besagte Vorrichtung außer dem Monofilament auch folgendes umfasst:
- einen halbtransparenten Schirm (1), auf dem eine Tafel (2) mit Bildern (3) des Fußes befestigt ist und der dazu geeignet ist, während der Untersuchung entweder vom Patienten oder seinem Assistenten in einer gewünschten Position zwischen dem Patienten und der ihn untersuchenden Person, bevorzugt auf den Knien des Patienten gehalten zu werden ; und
- eine Lampe (6, 7), bevorzugt eine Diode (8), die dazu geeignet ist, während der Untersuchung vom Patienten gehalten zu werden.

## Revendications

1. Dispositif pour l'examen du pieds d'un patient diabétique, comprenant au moins un mono filament destiné à effectuer une pression sur toute zone souhaitée sur le pieds du patient afin de déterminer si la pression et/ou l'endroit ou ladite pression s'exerce est alors identifiée par le patient ou non, **caractérisé en ce qu'**en plus dudit mono filament, ledit dispositif comprend, en outre:
- un écran (1) semi-transparent sur lequel est fixé un panneau (2) avec les images (3) des pieds et conçu pour être maintenu pendant l'examen, soit par le patient ou par son assistant dans une position souhaitée, entre le patient et la personne qui réalise l'examen, de préférence sur les genoux du patient ; et
- une lampe (6, 7), de préférence, une diode (8) qui est conçue pour être maintenue par le patient pendant l'examen.
